# EUROPEAN PATENT APPLICATION

(11) **EP 3 527 131 A1**
(43) Date of publication of application: **21.08.2019**
(21) Application number: 18157181.1
(22) Date of filing: 16.02.2018
(51) Int. Cl.: A61B 5/0484, A61B 5/00

(54) **COMPUTER-IMPLEMENTED METHOD AND APPARATUS FOR DETECTING AND PREDICTING THE NEURAL SIGNATURE OF DISORDERS OF EXECUTIVE FUNCTIONS AND WORKING MEMORY AND**

(71) Applicant: Centre Hospitalier Universitaire Vaudois (CHUV), 1011 Lausanne (CH)
(72) Inventor: Demonet, Jean-François, 74500 Neuvecelle (FR)
(74) Representative: KATZAROV S.A.

(57) **Abstract**

The present relates to a computer-implemented method for detecting and predicting cognitive pathologies of the brain based on a "Hold" and "Release" process measured by an electroencephalography machine or other neurophysiological and brain imaging equipment and methods, wherein the "Hold" and "Release" process comprises exposing a patient to successive stimuli, each stimuli meeting or not a specific criteria, the method comprising the use of trials made of series of items with trials involving designated targets made of the combination of item characteristics or categories as well as distracter trials in which items do not fulfill the designated criteria, with the following steps: instructing the patient which criteria shall meet an item and the target to detect in trials, exposing the patient with any trial of the Hold and Release process in a serial manner by categorizing the successive items, if a 1st item of a trial fulfills said specific criteria the patient shall hold his/her attention to the subsequent item of the current trial or if the 1st item does not fulfill said specific criteria the patient shall release his/her attention and disregard the subsequent item of the current trial, recording the patient's electrical activity in the form of electro-encephalographic data to measure continuously the neural signal as modulated by processing the HRA items, repetitively measuring the participant's brain activity over trials as in steps c and c' from the 1^{st} item onset inward, till the subsequent item onset, measuring in a similar way the neural activity over the inter-stimuli interval and then from the subsequent item onset till the end of the trial, and covering the participant's response recording the participant's response to the subsequent item, as in e', comparing said behavioral response and brain signal changes between Release and Hold conditions as well as between target and distracter sub-conditions amongst Hold trials, and deriving a global pattern of differential responses as well as differential indexes between those conditions and sub-conditions through a dedicated software and HRA-specific hardware devices implemented and connected to standard commercial EEG and ERP recording devices; for instance, indexes generated by the Hold-Release comparison between sustained negative variation observed after the 1^{st} item and the P3' observed after the subsequent item, g) statistic-based significant deviations of the observed values in a given participant from values in a reference population, when observed in the same date, in a congruent way and in at least two variants of HRA, are indicative of either an existing pathology of the brain affecting attention and working memory capacities or the risk for this neuropathology to develop on follow up changes over time of individual values as in g) are indicative of worsening or improvement of cognitive status either spontaneously or following any type of intervention.

## Description

### Technical Field

The present invention relates to a working memory measuring method and more particularly to a behavioral and neurophysiological method addressing the maintenance and disengagement of attention and the measuring of the same for the follow up of the disorders of executive functions and working memory.

Further, the present invention relates to a method for predicting cognitive function decay and brain degeneration. The invention relates to a computer-implemented method thereof. These methods are for example used in a clinical routine for helping taking therapeutic decisions and optimizing clinical care for each specific patient. In embodiments, the method is implemented in a software and dedicated hardware device connected to a clinical EEG machine for fast and automatic detection of these cognitive disorders and prediction of cognitive function degeneration.

### Background of the art

One generally knows that dementia affects a population of 47 million people worldwide; Alzheimer's disease accounts for more than half of the cases. Thus, WHO has made in 2017 dementia one of its priority objectives at the global level.

Age is the main risk factor; the number of patients is increasing with the constant trend of longer life expectancy, especially in the Western countries. Apart from aging, other factors, some genetic, others related to lifestyle (cardiovascular risk factors in particular) also play an important role. Besides, Alzheimer's disease and related brain pathologies generate health issues that go far beyond the yet very difficult question of dementia. Ageing-brain cognitive diseases such as Alzheimer's disease (AD) and related brain pathologies consist of slow, cumulative molecular processes that start developing in the human brain decades before the slightest trouble could be perceived in the affected person. This long silent phase is followed by a stage in which subtle difficulties may arise that are commonly confused with what is supposed to be the consequences of cerebral aging (e.g. prolonged reaction times or slower access to memories).

In addition to memory disorders that are the most well-known, these cognitive pathologies of the elderly brain are characterized initially by difficulties of decision-making and parallel management of multiple information, technically referred to as disorders of "executive" functions.

In view of the current situation, it is therefore a key objective to identify, among the many people with cognitive impairment, those who are most at risk of developing dementia later on. Many research programs aim at this overall objective but the high complexity of the related brain pathologies and the inability for a single technique to capture large enough accounting variance of pathology-to-normality distance, lead to the exploration of many dimensions such as: genetic characteristics, tissue biomarkers (eg cerebrospinal fluid, blood, tears, etc.), complex and costly brain imaging (e.g. PET scan), multiple cognitive tests, making such multi-dimensional screening in millions of people financially unbearable and unfeasible in practice, not even mentioning their ethical issues.

In this regard, a primary object of the invention is to address the above-mentioned problems and more particularly to provide a reliable method capable of detecting and quantifying a patient's attention and working memory capacities for the follow up of disorders of these functions.

### Summary of the invention

The above problems are addressed by the present invention.

This invention provides a method for exploring attention and working memory abilities as well as, for unravelling neural correlates of related cognitive disorders such as age-related cognitive decline and brain degeneration.

According to a preferred embodiment, the invention is a two-fold one.

First, it is based on a generic experimental cognitive paradigm, called Hold-Release (HR) that involves engagement versus disengagement of focused attention; it will hereafter be termed HRA (Hold-Release Attention) as a domain-general phenomenon; the latter may be affected by many brain diseases affecting cognition, hence the diagnosis properties of HRA especially in the early, incipient stages of age-related neurodegeneration.

Second, HRA yields a large amount of variants (e.g. depending on the type of external stimuli, whether verbal or non verbal, auditory or visual, etc.); each variant has the potential to explore domain-specific effects so that the fluctuations of attention to a specific repertoire of stimuli (e.g. words, faces, natural scenes, ...) and related working memory capacities generate engagement of specialized neural territories that can be explored using neurophysiological or brain imaging techniques (e.g. EEG, MRI, PET etc ..). HRA has therefore the capacity to explore specifically the consequences of neuropathologies targeting brain regions and functional domains such as language, vision or memory as domain-specific effects related to HRA.

The inventor previously designed and explored HRA using diverse brain mapping methods, from Positron Emission Tomography (PET) to Electroencephalography (EEG) and Magnetoencephalography (MEG) in healthy participants.

HRA yields engagement versus disengagement of focused attention and is based on the resolution of a two-step algorithm involving a double categorization in a serial presentation of pairs of items so that targets are distinguished from foils (more complex variants involving longer trials than pairs, for instance triplets or quadruplets were also used but are not described hereafter, although they are part of the invention).

The HRA method basically consists in a given trial in consecutively presenting a participant a pair of items (or stimuli, e.g. a word displayed on a screen), wherein each item shall meet a specified criteria or not, and measuring markers of attention and working memory performance, using behavioral responses (e.g. key press yielding accuracy scores and reaction times) and EEG recording.

According to this method, first the participant is instructed about the target pairs, i.e. those in which each item meets the required criterion, e.g., targets are pairs made of 2 words (such as table, chair, etc.), instead of pseudo-words (such as xhfvzds).

Once instructed, the participant proceeds with any trial by categorizing the 1st item presented, i.e. whether criterion 1 is met or not, i.e. whether It is a word or a pseudo-word.

If the 1st item is a pseudo-word, the participant can make his/her decision readily: the current trial, being for sure a distracter, is to be rejected; he/she can therefore release his/her attention and disregard the 2nd item: this case defines the Release condition.

If the 1st item does meet criterion 1 (e.g. it is a word), focused attention should then be maintained and the participant addresses whether the 2nd item too meets the required criterion 2 so that the current trial qualifies as a target, this case defines the Hold condition.

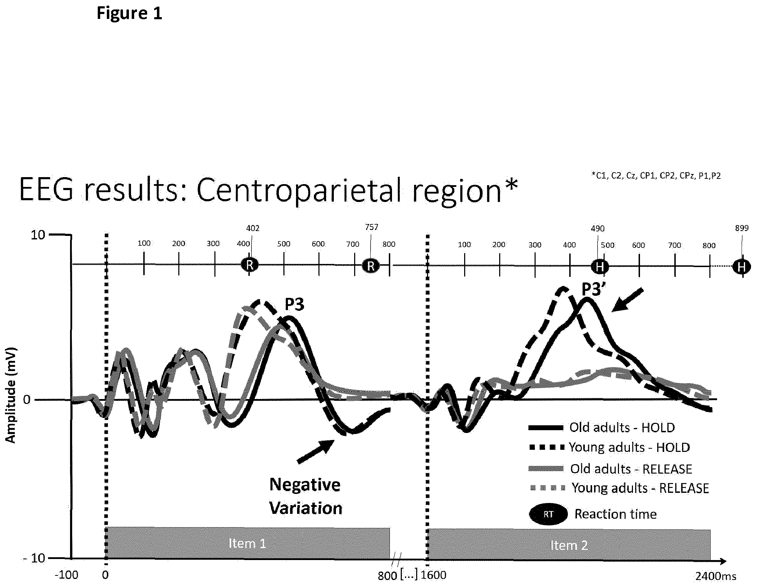

This is represented in figure 1 where upon coupled with brain responses recording such as ERP, Hold-release method allows for exploring not only response accuracy and timing (RT), but also the time course and amplitude of processes-related brain signals, especially selective attention and working memory load that are either focused on stimulus-specific processing ("Hold" condition) or withdrawn from it ("Release" condition).

For instance, in a variant of HR experiment involving a lexical decision task with combinations of words and/or pseudo-words in pairs, sustained negativity following the 1st item (so-called Negative Variation NV) and the P3' wave following the 2nd item represented in Figure 1 by the arrows were reliable markers of Hold condition; these markers witness engagement of selective attention and working memory load, as well as, for this P3', updating and registration processes.

Release trials/pairs are instead associated, after the first P3 generated by the 1^{st} item, with a positivity trend and the absence of P3' following the 2nd item.

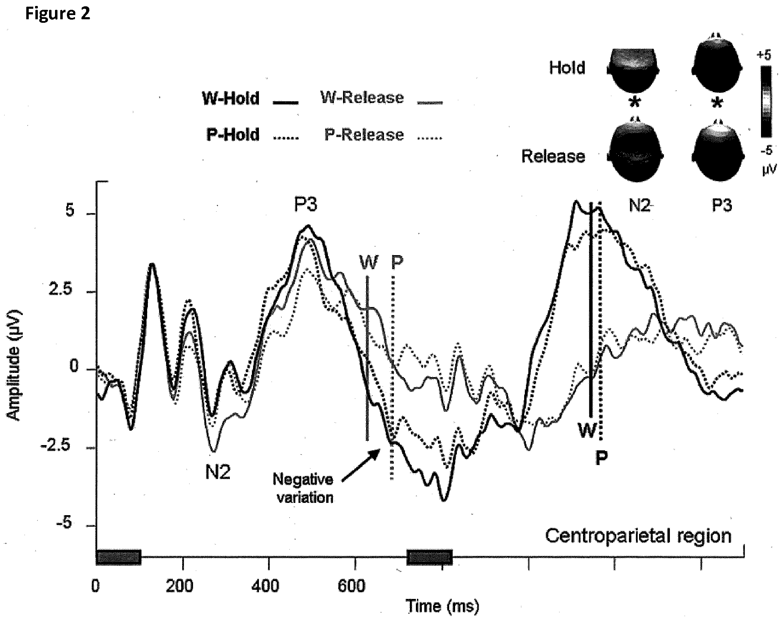

In figure 2, one can see ERPs recorded over centro-parietal region in 12 healthy participants. The figure summarizes findings observed in the same subjects in 2 variants of HRA where visually presented either pairs of words (plain curves) or pairs of pseudo-words (dotted curves) were defined as targets. Hold-related ERPs are shown in dark grey (W-Hold, P-Hold); Release ERPs are in light greys; the latter resulted from either pseudoword (W-Release) as 1st item when word-word was target or word when targets was pseudoword-pseudoword (P-Release). Arrows show sustained negativity following the item 1 (around 700 ms,) and P3' following the second item (around 400 ms) for both W-Hold and P-Hold.

In addition, we demonstrated that effects are independent of the lexical status of items. More recent experiments of ours revealed that the above described pattern for Hold- vs Release-generated ERPs is independent of the stimuli (whether verbal or other type of stimuli) involved in HRA. In a nutshell, HRA allows one to investigate the behavioral and neurophysiological correlates of the alternating engagement (Hold condition) and disengagement (Release condition) of attention resources that are allocated to stimulus-specific processing depending on task requirements. ERPs related to these typical HRA conditions are a domain-general phenomenon as independent of the type of stimuli involved in a given HRA variant.

While in an HRA variant related to lexical decision between words and non-words, we minimized working memory component as words were not semantically linked, we showed, in another, semantic-related HR variant, that higher working memory load was associated with increased activity in the lateral frontal cortex. Likewise, more recently, we explored using MagnetoEncephaloGraphy (MEG) the neural correlates of the maintenance of morpheme information in working memory during the Hold condition; lexical roots and suffixes of words (e.g. trad- / -er) were shown with the suffix presented as 1st item and the root as the 2nd, to increase working memory load. The task was to decide whether in a given pair, a suffix (e.g. -er, -ful, -ness, ...) matches a root (e.g. trad-, care-, smooth-) or not, so that root and suffix make a real word. This experiment addressed readily the unification process as one of the fundamental cognitive mechanisms that together with memory and control processes accounts for language comprehension and related executive functions.

In the present invention, we capitalize on our previous studies to show the clinical relevance of HRA-induced engagement of attention, working memory load and updating, as reflected in Hold by post 1^{st} item-sustained negativity (NV) and post 2^{nd} item-P3 (P3'), versus disengagement in Release. Although addressing domain-general processes, HRA has also a variety of applications for exploring domain-specific effects related to, for instance, language processes using dedicated HRA variants; the latter allow one for exploring the neural signature of such specific functional disorders in brain diseases, in particular age-related neurodegeneration..

According to a preferred embodiment of the present invention, HRA will therefore apply for instance to the early detection of long-term memory-related disorders induced by neurodegeneration targeting primarily the medial temporal cortex such as Alzheimer's disease. In this case HRA effects will be observed for both verbal and non-verbal (e.g. famous faces) variants and will be associated with an abnormal pattern of a recognition memory test coupled with these Alzheimer's-specific HRA variants (cf. infra).

Other applicative variants of HRA address other types of neurodegeneration, for instance, pathology affecting primarily language functions (e.g. primary progressive aphasias) or visual functions (Benson's syndrome, posterior cortical atrophy; as well as dementia with Lewy bodies). Other variants of HRA are also developed that are dedicated to traumatic brain injury (TBI) and to more focal brain diseases such as stroke; the same applies to psychiatric mood disorders and neurodevelopmental disorders such as schizophrenia, ADHD and dyslexia.

### Brief description of the drawings

Further particular advantages and features of the invention will become more apparent from the following non-limitative description of at least one embodiment of the invention which will refer to the accompanying drawings, wherein
Figure 1 represents averaged ERPs recorded over centro-parietal region in healthy, elder and young, participants who participated in an experiment using an HRA variant. Important features of the HRA pattern are shown, the negative variation observed after the 1^{st} item and the P3' observed after the 2nd item both in Hold condition (of note a first P3 is observed in any condition whether Hold or Release);
Figure 2 illustrates the similarity of HRA-related effects on ERPs whether target or distracters are words or pseudowords in a variant consisting in a lexical decision task, therefore showing the domain-general nature of HRA.

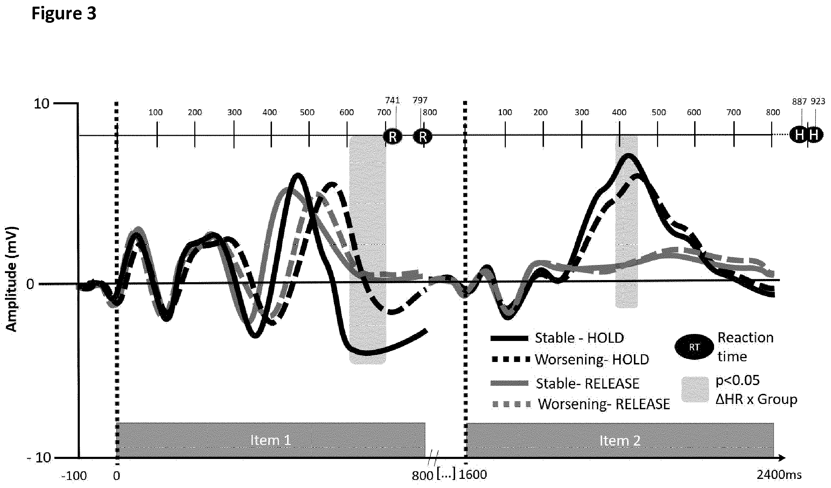

Figure 3 represents averaged ERPs recorded over centro-parietal region in an experiment using another HRA variant using semantic decision (as whether items are semantically related or not); here, the 65 elder subjects (mean age 74) who participated in HRA were divided in two groups as whether they show either stable verbal fluency performance over 2 consecutive assessments 5 years apart (N= 28) or worsened performance (N= 37). The average ERP curve recorded in the worsening participants shows, relative to the stable group, a striking decrease of the amplitude of the post-item1 Hold-related negative variation (that gets closer to the Release-related curve) and the same is true for the P3' observed in Hold condition following the item2.

### Detailed description of the invention

The present detailed description is intended to illustrate the invention in a non-limitative manner since any feature of an embodiment may be combined with any other feature of a different embodiment in an advantageous manner.

The HRA method of the present invention is a generic method as one can generate many different variants following the same principles while using varied type of stimuli (visual, auditory, and other sensory modalities), timing of presentation, type of responses such as manual, oral, oculo-motor; go-no-go versus A-B (obligatory) responses, in conventional or virtual reality environments (in the latter case, Hold responses evolve to "gathering" responses of virtual objects interesting to retain as a potential component of a target, while Release responses consist in discarding or throwing away irrelevant items), etc. all being within the scope of the present invention.

Stimuli are in either sensory modality, and HRA variants encompass a number of cognitive domains from various levels of language representations to memory processes as well as visual non-verbal perception (e.g. faces, natural or artefact objects, natural scenes) or other cognitive domains.

In HRA, target trials are in general defined by a specific perceptual or cognitive dimension of these items according to which items involved in these trials are categorized and unified; for instance, as exemplified above, targets may be pairs in which both the 1st and the 2nd item are real words, to be discriminated in turn from distracters; the latter are experimentally generated "pseudo-words" and may resemble existing words but do not qualify as an existing entry of the vocabulary of the participant's mother tongue.

Once instructed about target trials, i.e. those in which each item meets the required criteria, the participant proceeds with any trial in a serial manner, i.e. by categorizing the 1st item, as to the latter either fulfills criterion 1 or not.

If not (e.g. the 1st item is a pseudo-word), the participant can make readily his/her decision: the current trial, being for sure a non-target, is to be discarded; the participant can therefore release his/her attention and disregard the 2nd item: this case defines the Release condition.

In the case of Hold condition, the 1st item does meet criterion 1 (e.g. it is a word); focused attention should then be engaged and maintained to the point where the participant can address whether the subsequent item too meets the assigned criterion so that the current trial qualifies as a target.

Of note, while the below shown examples involve trials made of pairs, the same logic may be extended to longer stimulus sets (e.g. involving 3 or more items in series).

Coupled with brain responses recording (e.g. using Event-Related Potentials (ERP) from the EEG signal, or other techniques such as functional MRI, MEG, infrared multichannel recording etc ..), HRA allows for exploring not only response accuracy and response time (RT), but also the time course and amplitude of processes-related neural signals, especially those relating to focused attention and working memory that either allow for stimulus-specific processing ("Hold" condition) or are withdrawn from it ("Release" condition).

For instance, in the variant of HR experiment involving a lexical decision task (with combinations of words and/or pseudo-words in pairs, as mentioned above) sustained negativity following the 1st item and the P3' wave following the 2nd item (Figure 1 and 2) were reliable markers of Hold condition.

The sustained negativity following the 1st item has shown to witness engagement of selective attention and expectancy of the next stimulus.

Further recent evidence from the Inventor show that P3' is likely related to updating and registration processes.

Release trials (or pairs) were instead associated with positivity following the 1st item and the absence of P3' following the 2nd item.

Effects are independent of the lexical status of items (i.e. the pattern was very similar whether the designated target was pairs of words or pairs of pseudowords), a finding that has been confirmed by further experiments by the Inventor and collaborators using various types of stimuli whether verbal or non-verbal. It illustrates that the main pattern of HRA (Hold-related negative variation and P3') is domain-general and independent from the type of stimuli to be processed.

These typical features were recorded predominantly in central electrodes over the skull vertex (Pz, Cz, FCz) as well as in the neighboring lateral electrodes in both cerebral hemispheres.

In a nutshell, HR allows one to investigate the behavioral and neurophysiological correlates of the alternating engagement (Hold condition) and disengagement (Release condition) of attention resources that are allocated to stimulus-specific processing depending on task requirements.

This alternation has a profound functional significance as it relies to the various functional regimes that the brain develops in an awakened life as we keep switching from one task to another. In daily life, after attention resources have been instantly engaged in a given task, a switch to a different task or target with higher priority may become soon necessary. It may also turn out that the current environment is of low interest and it is preferable to pause for a while with external stimuli and the attention is redirected to inner sources. Such a "quieter" or internally driven neurofunctional regime has been identified as the brain "default mode" in which external stimuli tend to be disregarded while mental resources are re-focused to inner representations. The ability to seamlessly shift from one regime to another involves a complex interplay between competing, large-scale functional neural networks e.g. between the default mode network and other networks specialized in processing stimuli from the external world (e.g. speech signal). This apt switching across higher order functions is affected by a number of brain diseases and especially by neurodegeneration in the early stages of dementing conditions such Alzheimer's disease.

The present HRA invention focus on the ability to recognize, monitor and possibly diagnose this subtle dysfunction regime in the human brain, for instance in the early stages of neurodegeneration in elderly individuals.

Recently, this framework was used to design HRA tasks suitable for elderly participants using both nonverbal and verbal stimuli.

By comparison to young adults, consistent results were obtained in ca 100 elder participants; the latter showed delayed reaction times and delayed ERPs relative to results obtained in young adults, while the typical HR pattern is preserved as shown in Figure 1. For instance, Figure 1 shows that the young adults have an average reaction time for Release of about 402 ms while for the elder ones average reaction time is 757ms (see the R dots in the upperpart of figure 1). The same happens for the latency of P3' in Hold curve and the corresponding reaction times that are also set apart by ca. 300 ms in young relative to elder participants.

Further, preliminary evidence show that subtle deficits in attention and executive functions developing over time (i.e. ca 5 years) are reflected in behavioral measurements (Reaction Times) and, much more importantly, in HRA-generated ERPs so that the post-item1 sustained negativity and the P3' differ significantly in amplitude and latency, according to stability versus worsening of performance on attention-related tests such as verbal fluency tasks (see Figure 3).

Figure 3 clearly shows that upon HRA process, participants with stable performance over time show a profound negative variation when the first item meets the criteria, i.e. when they hold their attention, whereas in worsening participants in the same Hold condition, the amplitude of this component in much reduced when the first item meets the criteria, thereby suggesting a diagnosis of incipient brain degeneration.

More complex changes in ERPs are also associated with decline of other cognitive functions such as episodic memory. The invention covers the clinical use of all possible variants of the HRA paradigm mentioned above, as well as the use of advanced techniques for signal analyses and the use of complementary, multi-dimensional profiling that may increase the sensitivity and specificity of HRA for diagnosis and prognosis purposes.

This multi-dimensional profile involves
(i) other HRA-related features derived from changes in oscillatory EEG and MEG signal in a variety of frequency bands (from alpha to gamma),
(ii) The use of Artificial Intelligence techniques such as machine-learning (deep learning) and virtual-classifiers based analyses of HRA-generated neurophysiological signals
(iii) other brain imaging features (in the broadest sense) including functional connectivity analyses,
(iv) behavioral and cognitive phenotypic features that are associated with HRA, especially those related to disorders of attention, executive and memory functions in various brain diseases from developmental to neurodegenerative, traumatic, psychiatric and vascular stroke brain damage, and especially in the domain of the age-related cognitive decline (Alzheimer's disease (AD) and related dementing diseases). As regards cognitive phenotype, HRA involves especially post-hoc long term memory tests; these tests explore especially recognition memory test and may involve any type of item retrievable from any modality (visual, auditory, and others) of long-term memory (e.g. words, faces, artefactual or natural objects, landscapes and other scenes) that are used to form HRA item pairs (or longer stimulus sets in trials). Post-HRA recognition of the involved stimuli is explored as whether participants can distinguish between those stimuli they had to process (thereafter called OLD items) from never presented in HRA before (thereafter called NEW); performance measurements are based on this OLD/NEW recognition paradigm and otherwise derived behavioral features and allow for the exploration of the functional integrity of various parts of the mesial temporal cortex.
(v) specific genotype features (especially the genotype APOE4 associated with late-onset AD, as well as other genetic traits).
(vi) other peripheral physiological and biological markers (e.g. circulating biomarkers) that may complement and increase the diagnosis and prognosis accuracy of HRA-based method.

The HRA diagnosis and prognosis power is likely increased by deriving a unique profile involving HRA-derived characteristics combined with behavioral performance (for instance recognition memory performance) and genotypic features (e.g. including APOE4 for AD).

Notwithstanding other modalities of on-line brain activities recording (fMRI, functional near-infrared spectroscopy fNIRS), HRA provides mainly the user with typical changes in reaction times and Event-Related Potentials (ERPs) using surface (scalp) EEG recording under the engagement, maintenance and disengagement of attention that will allow the user to diagnose subtle or early disorders of executive, working memory and attention functions and to follow up longitudinal changes related to either clinically relevant, spontaneous changes of cognitive and behavioral abilities, as well as changes induced by any treatment (using drug compound or other therapeutic method) of the considered disorders.

HRA is a cost-effective screening tool to detect incipient age-related cognitive decline in asymptomatic volunteer participants that may be included in clinical studies including innovative treatments. Especially in the domain of the clinical neuroscience of ageing, by comparison to the existing methods, HRA is likely to be both more sensitive and easier to apply than conventional methods.

While a number of previous EEG studies pointed to the importance of P3 as a correlate of attention disorders in Mild Cognitive Impairment, these findings most likely do not capture early enough and with enough sensitivity changes that occur even before the MCI stage. We content that, compared to these previous studies, HRA bears additional diagnosis power for earlier deficit affecting attention and working memory capacities, especially the detection of changes in the post-1stitem sustained negativity.

Besides, PET scan and MRI were also used to tackle early-bird evidence of metabolic, structural or complex functional changes in the ageing brain. However, these techniques are, by far, less accessible, more invasive and dramatically more expensive than EEG. EEG/ERP signal recording necessary to reveal HRA is easy to obtain via only several electrodes (minimally one at vertex, and some in the periphery of the scalp) in a completely harmless way, so that wearable devices may be used for these measurements and the latter can be acquired virtually anywhere and in outside world conditions.

By comparison MRI and PET are heavy and highly expensive equipment that are in use only in relatively few medical Centers and University Hospitals; they use powerful electromagnetic fields and cannot be used easily in frequent medical conditions such as, for MRI, patients wearing cardiac pacemakers, vascular implants for MRI or for PET cannot be repeated easily owing to the limitation of allowed radioactivity doses. Finally, the cost of a standard MRI examination (ca. 800 €) is, at least, 16 times more expensive than an EEG and for PET the multiplicative factor is about 60 times.

Aside from its cost-effectiveness, HRA, a versatile experimental method, also allows researchers to explore in more details than with conventional ERPs, more complex neural correlates of attention-related fluctuations induced by Hold and Release conditions as a domain-general phenomenon; for instance, these neural signals consist of high-frequency oscillations and functional connectivity effects in the Alpha, Beta, Theta and Gamma bands explored with EEG or MEG, as well as their counterparts in functional MRI of near-infra red spectroscopy modalities such as changes in BOLD-like signals in task-induced and resting state conditions.

In addition, these changes in oscillatory regimes are expected to present with various topographical distributions, depending on the domain-specific effects tackled by specific variants of HRA, such as predominantly left-sided temporal distribution for language-related variants, right-sided temporal distribution for famous faces, or right-sided parietal in the case of visual-spatial variants; these and other specific distributions of signal changes are likely related to HRA, especially effects linked to loading of to-be-processed items in working memory and specific buffers depending on the type of contents and processes involved in variants of HRA. Such changes in HRA-induced oscillatory regimes are likely of diagnosis interest for specifically localized brain dysfunction, for instance verbal working memory effects in predominantly left-sided cortical lesions.

More in general, the invention comprises changes in Electroencephalographic (EEG), Magnetoencephalographic (MEG), fMRI or near-infrared spectroscopy signals related to HRA that will be demonstrated using machine learning and deep learning programs. These machine-learning methods allow for deciphering, at the single-individual and the single-event levels, neural responses related to either engagement or release of attention, as well as neural events related to memory load or retrieval associated with HRA. The invention covers both computer-implemented programs and hardware devices that are derived from the above described methods related to HRA; the user can therefore be provided with results significant in each and single subject who undergoes an HRA test following the use of software or hardware that are specific to the Invention. These individual results inform about attention and memory performance that together with other individual results such as genetic and non-genetic biological, cognitive or imaging factors are indicative of risk of brain diseases affecting cognition, e.g. neurocognitive disorders.

In sum, HRA consists in a new, harmless, cost-effective and widely useable neurofunctional tool to detect early evidence of clinically significant cognitive decline and to follow up these changes including objective evidence of positive effects of innovative treatments. These features make HRA a highly interesting method for any clinical team in the field of clinical neuroscience as well as any industrial company (e.g. pharmaceutical companies) that aim to monitor the effects of experimental treatments in an efficient and affordable way.

HRA applies to the diagnosis and follow up of disorders of executive functions, attention and working memory in humans in various clinical populations, especially in elder subjects affected by, or complaining about, age-related cognitive disorders. HRA can also be used as a surrogate marker of changes in the brain metabolism as a consequence of therapeutic intervention in the broadest sense (whether pharmacological or not). Other applications may be developed, also for monitoring the neural counterparts of therapeutic interventions, in younger patients be they affected by e.g. TBI, strokes, depression, neurodevelopmental pathologies, such as major psychiatric syndromes (e.g. schizophrenia), and in children affected by developmental learning disabilities, e.g. attention disorder or dyslexia.

While the embodiments have been described in conjunction with a number of others, it is evident that many alternatives, modifications and variations would be or are apparent to those of ordinary skill in the applicable arts. Accordingly, this disclosure is intended to embrace all such alternatives, modifications, equivalents and variations that are within the scope of this disclosure. This for example particularly the case regarding the different apparatuses which can be used.

## Claims

1. A computer-implemented method for detecting and predicting cognitive pathologies of the brain based on a "Hold" and "Release" process measured by an electroencephalography machine or other neurophysiological and brain imaging equipment and methods,
wherein the "Hold" and "Release" process comprises exposing a patient to successive stimuli, each stimuli meeting or not a specific criteria, the method comprising the use of trials made of series of items with trials involving designated targets made of the combination of item characteristics or categories as well as distracter trials in which items do not fulfill the designated criteria, with the following steps:
a) instructing the patient which criteria shall meet an item and the target to detect in trials,
b) exposing the patient with any trial of the Hold and Release process in a serial manner by categorizing the successive items,
c) if a 1st item of a trial fulfills said specific criteria the patient shall hold his/her attention to the subsequent item of the current trial or
c') if the 1st item does not fulfill said specific criteria the patient shall release his/her attention and disregard the subsequent item of the current trial,
d) recording the patient's electrical activity in the form of electro-encephalographic data to measure continuously the neural signal as modulated by processing the HRA items,
e) repetitively measuring the participant's brain activity over trials as in steps c and c' from the 1^{st} item onset inward, till the subsequent item onset,
e') measuring in a similar way the neural activity over the inter-stimuli interval and then from the subsequent item onset till the end of the trial, and covering the participant's response
e") recording the participant's response to the subsequent item, as in e'
f) comparing said behavioral response and brain signal changes between Release and Hold conditions as well as between target and distracter sub-conditions amongst Hold trials, and deriving a global pattern of differential responses as well as differential indexes between those conditions and sub-conditions through a dedicated software and HRA-specific hardware devices implemented and connected to standard commercial EEG and ERP recording devices; for instance, indexes generated by the Hold-Release comparison between sustained negative variation observed after the 1^{st} item and the P3' observed after the subsequent item
g) statistic-based significant deviations of the observed values in a given participant from values in a reference population, when observed in the same date, in a congruent way and in at least two variants of HRA, are indicative of either an existing pathology of the brain affecting attention and working memory capacities or the risk for this neuropathology to develop on follow up
h) changes over time of individual values as in g) are indicative of worsening or improvement of cognitive status either spontaneously or following any type of intervention.

2. The method according to claim 1, wherein step c) comprises repetitively measuring the participant's EEG activity or similar metabolic signal.

3. The method according to claim 1, wherein the Hold and Release process comprises exposing a patient to a pair of successive stimuli.

4. The method according to claim 1, wherein the brain imaging equipment and methods comprise MRI, MEG or near infrared spectroscopy.

5. The method according to claim 1, wherein the items are presented in a digitized format such as auditory, visual and the like.

6. The method according to claim 1, further comprising recording the participant's response by a dedicated equipment allowing for computerized recording of participant's choice of between possible responses, such as either target or distracter item, as well as reaction time.

7. The method according to claim 1, further comprising distributing over time both the trials (i.e. Stimulus Onset Asynchrony, SOA) and the items within a given trial (i.e. Inter Stimulus Interval, ISI) as a function of the type of participants and the pathology tested.

8. Medical or wearable devices adapted to carry out the method of claim 1.
